# EUROPEAN PATENT SPECIFICATION

(11) **EP 2 376 185 B1**
(45) Date of publication and mention of the grant of the patent: **30.03.2016**
(21) Application number: 09752262.7
(22) Date of filing: 11.11.2009
(51) Int. Cl.: A61N 1/36, A61N 1/378, A61N 1/375, H04R 25/00

(54) **MODULAR SPEECH PROCESSOR HEADPIECE**
MODULARES SPRACHPROZESSOR-KOPFTEIL
OREILLETTE MODULAIRE À PROCESSEUR VOCAL

(30) Priority: 04.03.2009 US 397982; 12.11.2008 US 113708 P; 20.12.2008 US 139567 P
(43) Date of publication of application: 19.10.2011
(73) Proprietor: Advanced Bionics AG, 8712 Stäfa (CH)
(72) Inventor: CRAWFORD, Scott, A., Castaic CA 91384 (US); LYNCH, Douglas, P., Shepherdstown WV 25443 (US); WOODS, Carla, Mann, Beverly Hills CA 90210 (US); GRIFFITH, Glen, A., Newbury Park CA 91320 (US)
(74) Representative: Schwan Schorer & Partner mbB
(86) International application number: PCT/US2009/064066
(87) International publication number: WO 2010/056768

(56) References cited:
- WO-A-2005/062668
- US-A- 5 949 895
- US-A1- 2002 032 472
- US-A1- 2004 073 275
- US-A1- 2005 245 991
- US-A1- 2006 190 059
- US-A1- 2007 053 534
- US-A1- 2007 282 394
- US-A1- 2008 002 834
- US-B1- 6 246 911

## Description

### BACKGROUND

In human hearing, hair cells in the cochlea respond to sound waves and produce corresponding auditory nerve impulses. These nerve impulses are then conducted to the brain and perceived as sound.

Hearing loss, which may be due to many different causes, is generally of two types: conductive and sensorineural. Conductive hearing loss typically occurs where the normal mechanical pathways for sound to reach the hair cells in the cochlea are impeded, for example, from damage to the ossicles. Conductive hearing loss may often be helped by using conventional hearing aids that amplify sounds so that acoustic information can reach the cochlea and the hair cells. Some types of conductive hearing loss are also amenable to alleviation by surgical procedures.

Many people who are profoundly deaf, however, have sensorineural hearing loss. This type of hearing loss can arise from the absence or the destruction of the hair cells in the cochlea which then no longer transduce acoustic signals into auditory nerve impulses. Individuals with sensorineural hearing loss may be unable to derive any meaningful benefit from conventional hearing aid systems no matter how loud the acoustic stimulus is. This is because the mechanism for transducing sound energy into auditory nerve impulses has been damaged. Thus, in the absence of properly functioning hair cells, auditory nerve impulses cannot be generated directly from sounds.

To overcome sensorineural deafness, cochlear implant systems or cochlear prostheses have been developed that can bypass the hair cells located in the cochlea by presenting electrical stimulation directly to the auditory nerve fibers. This leads to the perception of sound in the brain and provides at least partial restoration of hearing function. Most of these cochlear prosthesis systems treat sensorineural deficit by stimulating the ganglion cells in the cochlea directly using an implanted electrode or lead that has an electrode array. Thus, a cochlear prosthesis operates by directly stimulating normally transduce acoustic energy into electrical activity to the connected auditory nerve cells.

Prior to stimulating the nerve cells, the electronic circuitry and the electrode array of the cochlear prosthesis separate acoustic signals into a number of parallel channels of information, each representing a narrow band of frequencies within the perceived audio spectrum. Ideally, each channel of information should be conveyed selectively to a subset of auditory nerve cells that normally transmit information about that frequency band to the brain. Those nerve cells are arranged in an orderly tonotopic sequence, from the highest frequencies at the basal end of the cochlear spiral to progressively lower frequencies towards the apex.

A cochlear implant system typically comprises both an external unit that receives and processes ambient sound waves and a cochlear implant that receives data from the external unit and uses that data to directly stimulate the auditory nerve. A common configuration for a cochlear implant system thus involves internal components that are surgically implanted into the patient and external components that provide power and electrical signals representing environmental sound to the internal components. These external components typically include a Behind-the-Ear (BTE) processor worn on the ear or a body worn processor. These processors contain a microphone, batteries, and signal circuitry that processes the electrical signals generated by the microphone. The processors are connected to a headpiece by a cable. The headpiece receives the electrical signals through the cable and transmits them to the internal components.

In some cochlear implant systems, the cable or cables connecting the external components together can present some issues. For example, the cable may have to be routed through clothing or accommodated during hair styling. The cable may be snagged, pulled on, or tangled, causing the headpiece to fall off. Additionally, cables are considered unattractive by many patients and are susceptible to failure due to bending.

US 2008/0002834 discloses a cochlear implant system according to the preamble of claim 1.

US 200610190059 A1 relates to a cochlear implant system according to the preamble of claim 11 , comprising a magnet-less implanted cochlear stimulator and a headpiece-less BTE external sound processor. A connectivity module may be connected to the BTE module via a cable connection, with the connectivity module including a battery. Alternatively, the BTE module may be provided with attachable modules, such as a zinc air battery module or a lithium ion battery module.

### BRIEF DESCRIPTION OF THE DRAWINGS

The accompanying drawings illustrate various embodiments of the principles described herein and are a part of the specification. The illustrated embodiments are merely examples and do not limit the scope of the claims.
FIG. 1A illustrates an exemplary cochlear implant system according to principles described herein.
FIG. 1B illustrates a portion of an exemplary cochlear implant system according to principles described herein.
FIG. 2 is a functional block diagram of an exemplary sound processor and implantable cochlear stimulator according to principles described herein.
FIG. 3 illustrates a schematic structure of the human cochlea highlighting elements according to principles described herein.
FIG. 4 is a perspective view of a modular speech processor headpiece, according to the present invention.
FIG. 5 is a perspective view of a modular speech processor headpiece, according to one embodiment of the present invention.
FIG. 6 shows a modular speech processor headpiece interfacing with internal components of a cochlear implant system, according to one embodiment of the present invention.
FIG. 7 is a perspective view of modular speech processor headpiece, according to the present invention.
FIGS. 8A and 8B are a top and side view, respectively, of a modular speech processor headpiece, according to the present invention.
FIG. 8C is a top view of an illustrative modular speech processor headpiece showing the motion of a latching component, according to principles described herein.
FIGS. 9A and 9B are a top and a side view, respectively of a modular speech processor headpiece, according to the present invention.
FIG. 10A is an exploded perspective view of an exemplary modular speech processor headpiece.
FIG. 10B is a cross-sectional side view of a portion of an exemplary modular speech processor headpiece.
FIG. 11 illustrates magnetic flux surrounding an induction coil and a retention magnet in an exemplary modular speech processor headpiece.

Throughout the drawings, identical reference numbers designate similar, but not necessarily identical, elements.

### DETAILED DESCRIPTION

The invention relates to a system as defined in claim 1 and a system as defined in claim 11, respectively.

The dependent claims define preferred embodiments. Aspects, embodiments, examples or illustrations of the present disclosure that do not fall within the scope of the appended claims are not part of the present invention.

As mentioned above, individuals with hearing loss can be assisted by a number of hearing assistance devices. These assistive devices are typically worn regularly and over a significant period of each day. Consequently, any such hearing assistance device should be robust and reliable. Additionally, the hearing assistance devices should be visually unobtrusive and not unduly restrict the user's activities. As explained above, cochlear implant users typically must wear at least two separate external units, a processor and a headpiece, that are connected by a cable.

The processor in a conventional system may be a Behind-The-Ear (BTE) processor or a body worn processor. A BTE processor typically uses a hook which attaches over the top of the outer ear and holds the BTE processor in place behind the ear of the user. The BTE processor contains a microphone, battery, and electronics. A cable attaches the BTE processor to the headpiece and conveys data signals and power to the headpiece. The headpiece is typically held in place by magnetic forces generated by a surgically implanted magnet which is a part of the internal cochlear implant.

A body worn processor is typically worn by attaching the processor to an article of clothing worn by the user. For example, a body worn processor may be tucked into a pocket or attached to a lapel. The body worn processor does not have the severe size and weight constraints that are associated with a BTE processor. Consequently, the electronics and battery capacity of the body worn processor can be significantly greater than BTE processors. Like the BTE processor, a cable attaches the body worn processor to the headpiece.

As mentioned above, the cable or cables connecting the external components together can be difficult to manage. For example, when a child wears a cochlear implant, the parent may have to take additional care in dressing the child and restrict some activities the child would otherwise enjoy to prevent the cable from being snagged, pulled on, tangled, or broken. Additionally, the processor and cable can be visually distracting and are considered unattractive by many patients. For some patients, the BTE unit can be uncomfortable, particularly those who are sensitive to heavy objects hanging from their ears.

Accordingly, the present specification addresses these issues by describing a modular speech processor headpiece that combines the external components of the cochlear system into a single modular speech processor headpiece that is worn directly over the surgically implanted antenna. The modular speech processor headpiece is a head mounted, external component which provides a stand-alone support for the functionalities of the implanted components. The modular speech processor headpiece eliminates the need for a separate body worn processor or BTE processor and the connecting cable. Consequently, the modular speech processor headpiece reduces the complexity of wearing and using a cochlear implant. The cochlear implant system no longer requires a cable or a separate processor unit. This eliminates the need to route the cable through clothing or hair and additionally eliminates the possibility of snagging the cable. Additionally, the modular speech processor headpiece can be significantly less visually intrusive and more user friendly. The modular nature of the integrated cochlear implant headpiece may allow for other devices to communicate with and/or be attached to the integrated cochlear implant headpiece to provide additional functionality. However, the integrated head speech processor headpiece piece is configured to provide the basic functionality for the operation of the cochlear implant as a stand alone unit.

In the following description, for purposes of explanation, numerous specific details are set forth in order to provide a thorough understanding of the present systems and methods. It will be apparent, however, to one skilled in the art that the present systems and methods may be practiced without these specific details. Reference in the specification to "an embodiment," "an example," or similar language means that a particular feature, structure, or characteristic described in connection with the embodiment or example is included in at least that one embodiment, but not necessarily in other embodiments. The various instances of the phrase "in one embodiment" or similar phrases in various places in the specification are not necessarily all referring to the same embodiment. Also, as used in the specification and appended claims the term "headpiece" refers to a component that is worn on the user's head in proximity to an internal antenna, as opposed to a BTE processor or a body worn processor that are worn on the ear or on an article of clothing.

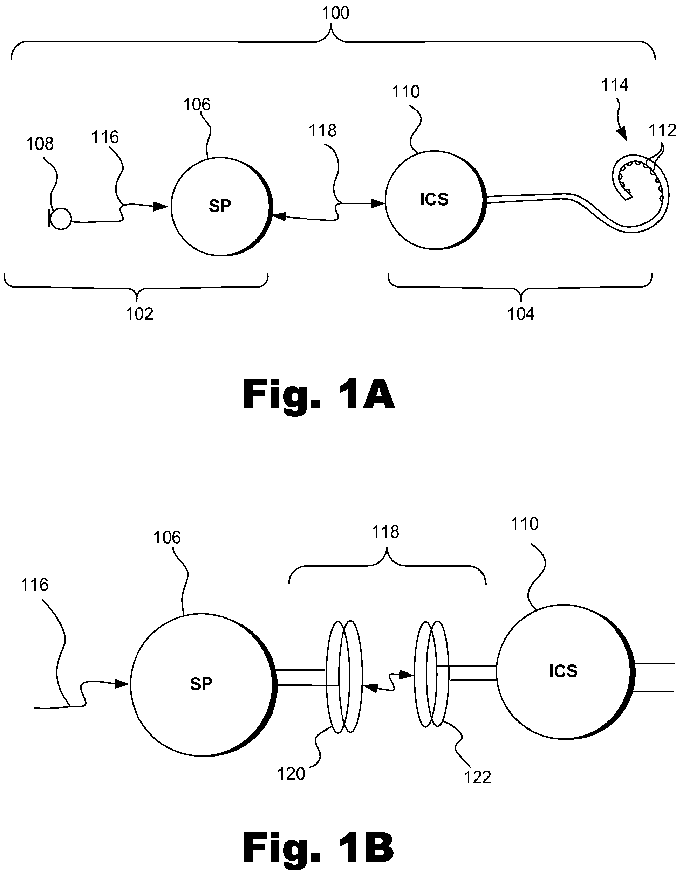

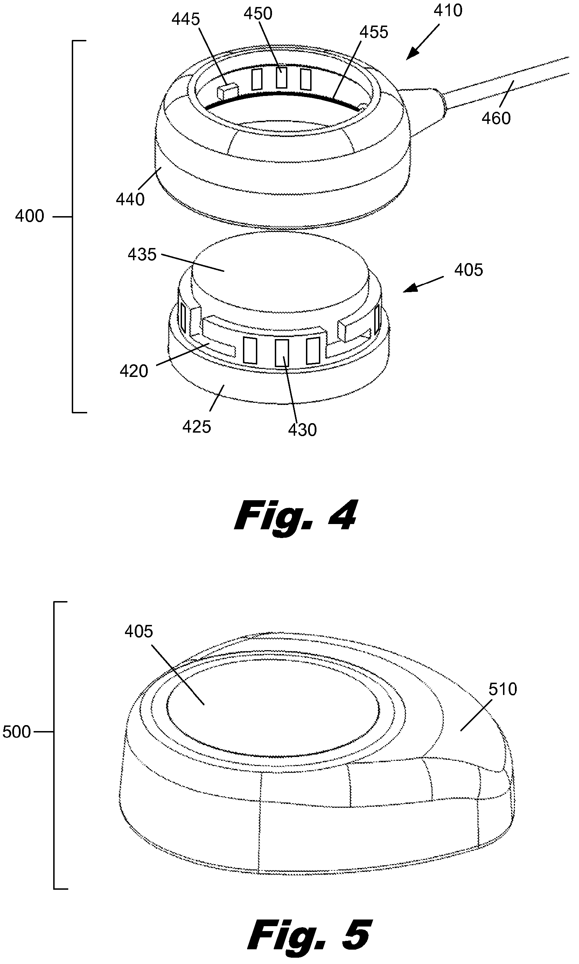

FIG. 1A illustrates an exemplary cochlear implant system 100. The cochlear implant system 100 of FIG. 1A includes a modular speech processor headpiece (or "modular headpiece") 102 and a cochlear stimulation device 104. The modular headpiece 102 may include a sound processor 106, a microphone 108, and/or additional circuitry as best serves a particular application. The cochlear stimulation device 104 may include an implantable cochlear stimulator 110, a number of electrodes 112 disposed on an electrode lead 114, and/or additional circuitry as best serves a particular application. The components within the modular headpiece 102 and the cochlear stimulation device 104 will be described in more detail below. For example, the modular aspects of the present systems are discussed below with reference to FIGS. 4-9B. Moreover, detailed descriptions of various aspects of cochlear implant systems but are not necessary for an understanding of the present inventions, are disclosed in U.S. Patent Nos. 6,219,580; 6,272,382; and 6,308,101.

The microphone 108 of FIG. 1A is configured to sense audio signals and convert the sensed signals to corresponding electrical signals. In some examples, the audio signal may include speech. The audio signal may additionally or alternatively include music, noise, and/or other sounds. The electrical signals are sent from the microphone 108 to the sound processor 106. The microphone 108 may be connected directly to, or integrated with, the modular headpiece 102. Alternatively, the microphone 108 may be connected to the modular headpiece 102 by way of a communication link 116 or a cable. The sound processor 106 processes the converted audio signals from the microphone in accordance with a selected sound processing strategy to generate appropriate stimulation parameters for controlling the implantable cochlear stimulator 110. These stimulation parameters may specify or define the polarity, magnitude, location (i.e., which electrode pair or electrode group receive the electrical stimulation), stimulation rate, timing (i.e., when the electrical stimulation is to be applied to a particular electrode pair), spectral tilt, and/or any other characteristic of the electrical stimulation that is generated by the implantable cochlear stimulator 110.

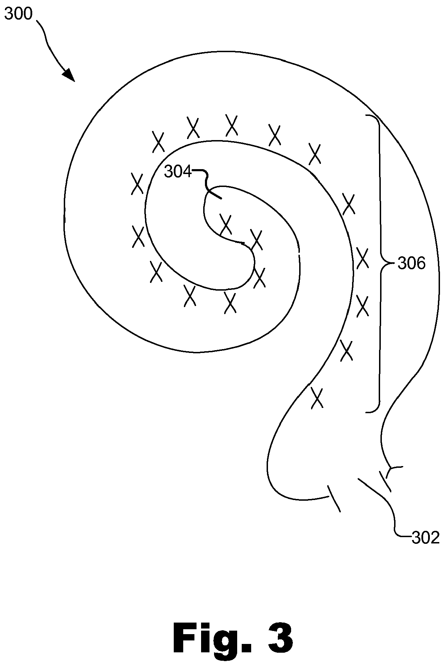

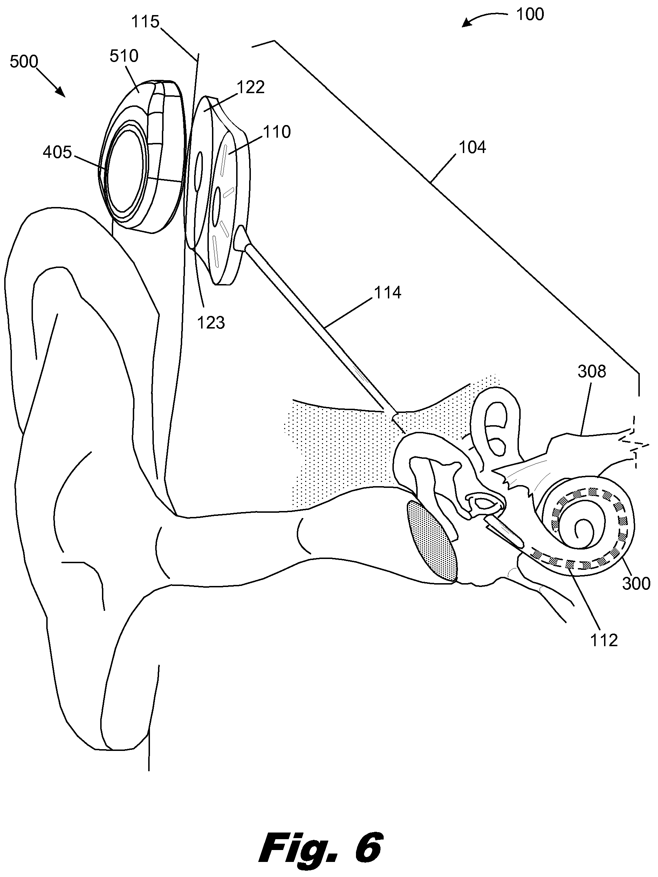

The exemplary electrode lead 114 shown in FIG. 1A is configured to be inserted within a duct of a cochlea. As shown in FIG. 1A, the electrode lead 114 includes a multiplicity of electrodes 112, for example, sixteen electrodes, spaced along its length. It will be understood, however, that any number of electrodes 112 may be disposed on the electrode lead 114. The electrode lead 114 may be substantially as shown and described in U.S. Patent Nos. 4,819,647 or 6,218,753, each of which is incorporated herein by reference in its respective entirety. As will be described in more detail below, and referring to FIG. 3 and 6, electronic circuitry within the implantable cochlear stimulator 110 is configured to generate and apply electrical stimulation to one or more stimulation sites within the cochlea 300 via selected stimulation channels (i.e., pairs or groups of the individual electrodes 112) in accordance with a specified stimulation strategy defined by the sound processor 106, and neural electrical signals are conducted from the cochlea 300 to the auditory cortex by the auditory nerve 308.

In some examples, the sound processor 106 and the microphone 108 comprise an external portion of the cochlear implant system 100, and the implantable cochlear stimulator 110 and the electrode lead 114 comprise an implantable portion of the system 100 that is implanted within a patient's body. In alternative examples, one or more portions of the sound processor 106 are included within the implantable portion of the cochlear implant system 100.

The exemplary implantable cochlear stimulator 110 and the sound processor 106 may be communicatively coupled via a suitable data or communication link 118, such as a telemetry communication link, as will be described in more detail below. It will be understood that the data communication link 118 may include a bi-directional communication link and/or one or more dedicated uni-directional communication links. In some examples, the external and implantable portions of the cochlear implant system 100 may each include one or more inductive coils (discussed below) configured to transmit and receive power and/or control signals via the communication link 118. The control signals may include, for example, the magnitude and polarity of electrical stimulation representing a sensed audio signal. The external coil may also transmit power from the external portion to the implantable portion of the cochlear implant system 100. Power transmitted to the implantable portion may be used to operate the implantable portion.

FIG. 1B illustrates a portion of an exemplary cochlear implant system showing a communication link 118 comprising a telemetry communication link that may be used in accordance with the present systems and methods. As illustrated in FIGS. 1A and 1B, the modular speech processor headpiece 102 of the cochlear implant system 100 may include an external induction coil 120 and the cochlear stimulation device 104 may include an implantable induction coil 122. The external induction coil 120 may be communicatively coupled to the sound processor 106 and the implantable induction coil 122 may be communicatively coupled to the implantable cochlear stimulator 110. It should be noted here that the present inventions are not limited to electromagnetic induction based communication devices. Other exemplary communication devices includes, but are not limited to, radio frequency communication devices, optical communication devices, and any other wireless communication devices.

The external induction coil 120 and the implantable induction coil 122 may include any suitable type of coil capable of generating and/or receiving an electro-magnetic field. For example, the external induction coil 120 and the implantable induction coil 122 may each include a metallic wire or tube wound in a coiled or otherwise looped configuration. An alternating current may be directed from the sound processor 106 through the external induction coil 120, thereby generating a magnetic field surrounding the external induction coil 120. The external induction coil 120 may be positioned near the implantable induction coil 122 such that the implantable induction coil 122 is positioned at least partially within the magnetic field generated by the external induction coil 120. The magnetic field generated by the external induction coil 120 may cause an electric current to be generated in the internal induction coil 120. The electric current generated in the internal induction coil 120 may be directed from the internal induction coil 120 to the implantable cochlear stimulator 110. Accordingly, an electric current generated by the sound processor 106 may be transferred to the implantable cochlear stimulator 110 through the communication link 118 comprising the external induction coil 120 and the implantable induction coil 122.

The communication link 118 may function as a telemetry link between the sound processor 106 and the implantable cochlear stimulator 110. For example, the external induction coil 120 may transmit one or more telemetry signals to the implantable induction coil 122 by generating a telemetry magnetic field as electric current is passed through the external induction coil 120. The telemetry magnetic field generated by the external induction coil 120 may produce an electric current in the implantable induction coil 122, as described above. The current generated in the implantable induction coil 122 by the telemetry magnetic field generated by the external induction coil 120 may be used to transfer signals representative of data and/or other information to the implantable cochlear stimulator 110 and/or may be used to transfer power to the implantable cochlear stimulator 110.

In some examples, the communication link 118 may be used to transmit telemetry signals from the implantable cochlear stimulator 110 to the sound processor 106. For example, data acquired by the electrodes 112 and/or status indicators generated by the cochlear stimulator 112 may be transmitted to sound processor 106 via the communication link 118. To this end, implantable induction coil 122 may transmit telemetry signals to the external induction coil 120 by generating a telemetry magnetic field. The implantable cochlear stimulator 110 may cause a current to flow through the implantable induction coil 122 to generate the telemetry magnetic field. The external induction coil 120 may be positioned at least partially within the telemetry magnetic field generated by the implantable induction coil 122. The magnetic field may cause an electric current to be generated in the external induction coil 120. The current generated in the external induction coil 120 may be used to transfer data and/or other signals to the sound processor 106.

The communication link 118 may include more than one external induction coil 120 and/or more than one implantable induction coil 122. For example, a first external induction coil and a first implantable induction coil may be used to transfer data and/or power from the sound processor 106 to the implantable cochlear stimulator 110. A second external induction coil and a second implantable induction coil may be used to transfer data from the implantable cochlear stimulator 110 to the sound processor 106.

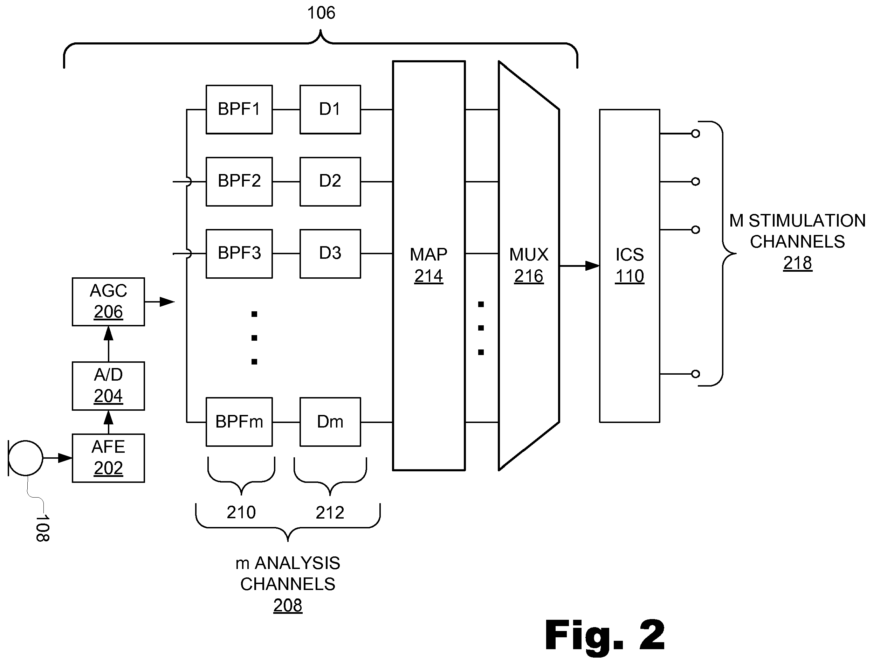

FIG. 2 is a functional block diagram of an exemplary sound processor 106 and implantable cochlear stimulator 110. The functions shown in FIG. 2 are merely representative of the many different functions that may be performed by the sound processor 106 and/or the implantable cochlear stimulator 110.

As shown in FIG. 2, the microphone 108 senses an audio signal, such as speech or music, and converts the audio signal into one or more electrical signals. These signals are then amplified in audio front-end (AFE) circuitry 202. The amplified audio signal is then converted to a digital signal by an analog-to-digital (A/D) converter 204. The resulting digital signal is then subjected to automatic gain control using a suitable automatic gain control (AGC) function 206.

After appropriate automatic gain control, the digital signal is then processed in one of a number of digital signal processing or analysis channels 208. For example, the sound processor 106 may include, but is not limited to, sixteen analysis channels 208. Each analysis channel 208 may respond to a different frequency band of the sensed audio signal due to a series of band pass filters 210.

As shown in FIG. 2, each of the m analysis channels 208 may also include an energy detection stage (D1-Dm) 212. Each energy detection stage 212 may include any combination of circuitry configured to detect the amount of energy contained within each of the m analysis channels 208. For example, each energy detection stage 212 may include a rectification circuit followed by an integrator circuit.

After energy detection, the signals within each of the m analysis channels 208 are forwarded to a mapping stage 214. The mapping stage 214 is configured to map the signals in each of the m analysis channels 208 to one or more of M stimulation channels 218. In other words, the information contained in the m analysis channels 208 is used to define the electrical stimulation pulses that are applied to the patient by the implantable cochlear stimulator 110 via the M stimulation channels 218. As mentioned previously, pairs or groups of individual electrodes 112 may make up the M stimulation channels 218.

In some examples, the mapped signals are serialized by a multiplexer 216 and transmitted to the implantable cochlear stimulator 110. The implantable cochlear stimulator 110 may then apply electrical stimulation via one or more of the M stimulation channels 218 to one or more stimulation sites within the duct of the patient's cochlea. As used herein, the term "stimulation site" will be used to refer to a target area or location to which the electrical stimulation is applied. For example, a stimulation site may refer to any location within a region of auditory nerve tissue.

FIG. 3 illustrates a schematic structure of the human cochlea 300. As shown in FIG. 3, the cochlea 300 is in the shape of a spiral beginning at a base 302 and ending at an apex 304. Within the cochlea 300 resides auditory nerve tissue 306, which is denoted by Xs in FIG. 3. The auditory nerve tissue 306 is organized within the cochlea 300 in a tonotopic manner. Low frequencies are encoded at the apex 304 of the cochlea 300 while high frequencies are encoded at the base 302. Hence, each location along the length of the cochlea 300 corresponds to a different perceived frequency. A cochlear prosthesis may therefore be implanted within a patient with sensorineural hearing loss and configured to apply electrical stimulation to different locations within the cochlea 300 to provide the sensation of hearing. For example, electrode lead 114 may be disposed within the cochlea 300 such that electrodes 112 contact auditory nerve tissue 306 within the cochlea 300. Electrical stimulation may be applied by the electrodes 112 to the auditory nerve tissue 306.

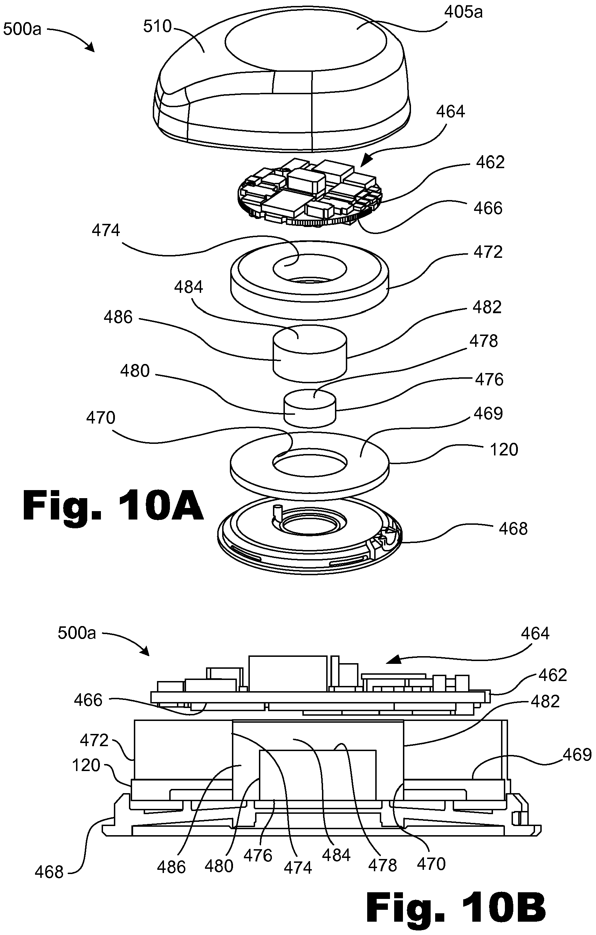

FIG. 4 is a perspective view of a modular speech processor headpiece 400 in accordance with one embodiment of a present invention. The modular speech processor 400 includes a core 405 and a modular ring 410. The core 405 contains all of the external electronics such as the power conditioning electronics, signal processors, filters, amplifiers, receivers, switches, memory, etc. For example, the core 405 includes the sound processor 106, microphone 108, coil 122, and other circuitry illustrated in FIGS. 1A-2. Many of the electronic components (e.g. the processors) may be carried on a circuit board. The base 425 and other portions of the core 405 may be substantially hollow to receive the electronics, as is discussed below with reference to FIGS. 10A and 10B.

Additionally, the modular speech processor 400 may contain a retention magnet or magnetic material, such as a magnet or magnetic material within or adjacent to the base 425, which is attracted to a magnet 123 (FIG. 6) or magnetic material within the implantable cochlear stimulator 110 adjacent the coil 122 (FIGS. 1A and 1B). The attraction between the base 425 and the magnet or magnetic material within the implantable cochlear stimulator 110 holds the coil 120 of the headpiece 400 in place over the implantable cochlear stimulator coil 122.

The upper surface 435 may contain various elements such as microphone ports and visual indicators. According to one embodiment, a microphone port allows for a microphone within the core 405 to sense sound pressure waves. Various visual indicators, such as one or more light emitting diodes, could also be included on the upper surface 435 to communicate information regarding the function of both internal and external components of the cochlear implant system, such as battery status, the selected program, sensitivity or volume information, and communication status between the headpiece 400 and implantable cochlear stimulator 110.

According to one embodiment, the core 405 is configured to receive various modular rings. In this embodiment, the modular rings may connect to core 405 by sliding tabs into slots 420 and then rotating the modular ring about the core 405 to lock the modular ring into place. The core 405 may contain a number of electrical contacts 430 which engage with matching electrical contacts on a modular ring, thereby allowing for electrical power and/or signals to be transmitted between the modular ring the core 405.

According to one embodiment, the modular ring 410 includes a housing 440 and a cable 460 connected to the housing 440. On the inner surface of the modular ring 410, a number of tabs 445 are configured to be received by the corresponding slots 420 in the core 405. Similarly, a number of contacts 450 on the inner surface of the modular ring 410 are configured to make electrical contact with the contacts 430 on the outer surface of the core 405. According to one embodiment, the modular ring 410 may also contain a number of o-rings 455 which seal against one or more surfaces of the core 405 to prevent contaminants from interfering with the electrical connections or otherwise disrupting the function of the core 405 and modular ring 410.

When the modular ring 410 includes a cable 460, the modular ring 410 may be attached to the core 405 for programming, to provide access to a long term power source, and/or to allow the user to access various accessories such as an external microphone, Bluetooth® receiver, or other accessories. For example, during programming or fitting the device by an audiologist, a corded modular ring 410 could be attached to the core 405 and connected to a programming computer. Additionally, a corded modular ring 410 may allow the user to access various conventional battery sources such as a battery pack. By accessing an external power source through the corded modular ring 410, the size and weight of the headpiece can be minimized. Additionally, the external power source may have increased battery capacity when compared with conventional BTE units.

In other situations, a modular ring that includes cable may not be the most desirable configuration. The modular nature of the core allows for any one of a number of alternative modular rings or other modular components to be attached to the core. FIG. 5 is a perspective view of a modular speech processor headpiece 500 in which the power source is integrated into a modular ring 510. As described above, the modular ring 510 fits over and around the core device and rotates to a locked position for security. In addition, the ring-to-core connection may provide a waterproof seal.

By way of example and not limitation, the modular ring 510 may contain a number of power sources, such as conventional lithium ion batteries, polymer lithium batteries, or zinc air batteries. Polymer lithium batteries operate using the same chemistry as conventional lithium ion batteries but contain the lithium-salt electrolyte within a solid polymer composite rather than a rigid metal case. Consequently, polymer lithium batteries can be lighter, more energy dense, and less vulnerable to physical damage. Further, polymer lithium batteries can be specifically shaped to fit the device it will power. Zinc air batteries operate by the oxidation of zinc with atmospheric oxygen. Zinc air batteries have high energy densities and are relatively inexpensive to produce. However, to operate, zinc air batteries must have direct exposure to the atmosphere which creates challenges in using these batteries in sealed systems.

Additionally or alternatively, the modular ring may contain any of a number of alternative accessories, such as radio frequency RF receivers or Bluetooth® receivers. These accessories can directly link the cochlear implant system to sound sources, reducing interference by other noise sources. For example, in an educational setting, teacher may wear a wireless microphone which transmits the teacher's voice over a radio frequency directly to a receiver contained within the modular ring. Similarly, a Bluetooth® receiver could be connected to a stereo, cell phone, or other audio source.

FIG. 6 is a diagram showing the modular speech processor headpiece 500 interfacing with internal components of a cochlear implant system, e.g. the above-described cochlear stimulation device 104. As mentioned above, the modular speech processor headpiece 500 may contain a core 405 and a modular ring 510 that contains a battery power source. This configuration consolidates all of the external components of the cochlear implant system into a single modular speech processor headpiece 500. This eliminates cables connecting the components together and the associated problems of routing the cables through clothing or the cable being snagged, pulled on, or tangled, causing the headpiece to fall off. Additionally, the modular speech processor headpiece 500 is more discrete than systems with multiple components. For example, the modular speech processor 500 may be completely covered by the user's hair or hat. Further, the modular speech processor headpiece 500 may be more robust than multiple component configurations. The modular speech processor headpiece 500 is easier to seal and eliminates cables which are susceptible to failure.

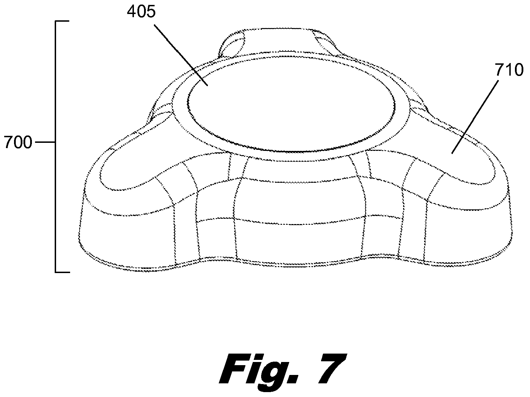

A number of alternative embodiments can be created by attaching various modular rings to the core. FIG. 7 is a perspective view of a modular speech processor headpiece 700 in which a modular ring 710 is attached to the core 400. According to one exemplary embodiment, the modular ring 710 has a number of lobes, each of which is configured to receive a one or more button batteries. In an alternative embodiment, a lithium polymer battery can be shaped to be received by the modular ring 710.

In a number of embodiments described above, the top and bottom surfaces of the core 405 are exposed. There are a number of advantages to exposing the top and bottom of the core 405. For example, the core 405 can be manipulated by grasping the exposed top and bottom of the core 405, for example, between a thumb and forefinger. This allows the core 405 to be easily grasped and rotated to attach or detach the modular ring 410, 510, 710. The top and bottom of the core 405 may have a number of features or characteristics which facilitate grasping and manipulating the core 405. By way of example and not limitation, the top and/or bottom of the core 405 may be textured or contoured to provide a better gripping surface.

Additionally, there may also be a number of other functional advantages to exposing the top and bottom of the core 405. By exposing all or a portion of the top surface of the core, visual indicators can be placed on the top surface of the core 405. These visual indicators may communicate the status of the cochlear implant or attached battery. The top surface of the core 405 may also have a microphone port which needs to have a direct channel to the exterior environment. By exposing the bottom portion of the core, the transmitter within the core can better communicate with the underlying antenna.

The modular ring configurations described above are only one illustrative embodiment of modular components which connect to a core. There are a variety of other configurations which provide modularity and customization of a cochlear implant. By way of example and not limitation, a variety of modular components may be attached to the core without entirely enclosing the perimeter of the core.

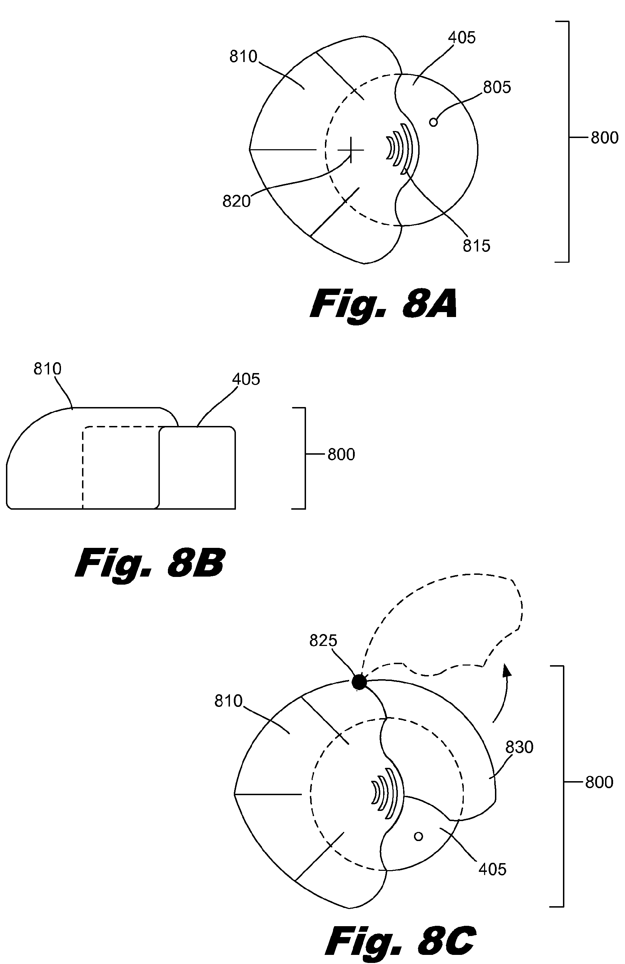

FIGS. 8A and 8B are a top view and a side view, respectively, of a modular speech processor headpiece 800 with an alternative geometry. Similar to other modular speech processor headpieces discussed above, this headpiece 800 combines all of the external components of a cochlear implant into a core 405. A modular component 810 attaches to one side of the core 405. The modular component 810 may serve a variety of functions, including providing power to the core 405. Additionally, the modular component 810 may contain a variety of receivers. By way of example and not limitation, a receiver within the modular component 810 may include Bluetooth®, radio frequency, 802.11, or other capabilities. The core 405 may also contain an internal microphone which accesses external sound waves through a microphone port 805.

The core 405 also includes an attachment means centered about the "+" 820. As previously mentioned, the attachment means may be made up of a magnetic attraction between a surgically implanted magnetic component and a second magnetic component within the core 405. For example, a magnet may be surgically implanted and a ferromagnetic material may be contained within the core 405 or vice versa. Additionally, two magnets may be used, one within the core 405 and one surgically implanted with the coil 122 (FIGS. 1A and 1B) or other antenna. When properly oriented, the use of two magnets can provide superior attractive force and/or allow the size of the magnetic components to be reduced.

To attach the headpiece 800, the headpiece 800 is simply brought into proximity with surgically implanted magnetic component. The attraction between the two magnetic components then centers the headpiece 800 over the coil 122 (FIGS. 1A and 1B) or other antenna and holds the headpiece 800 in place.

The location of the attachment means can be positioned within the core 405 such that when the modular component 810 is attached to the core 405, the headpiece 800 is balanced about the attachment point 820. By balancing the headpiece 800 about the attachment point 820 i.e. positioning the attachment point at the center of mass of the combined headpiece, the tendency of the headpiece 800 to rotate about the attachment point 820 when undergoing linear acceleration or vibration is significantly reduced or eliminated. By reducing the tendency of the headpiece 800 to rotate, the headpiece 800 becomes more comfortable to wear, especially for active individuals. Additionally, the abrasion of skin which is interposed between the headpiece 800 and the antenna 185, FIG. 6 can be reduced when the motion of the headpiece 800 is reduced.

The illustrative configuration shown in FIGS. 8A and 8B allows the core and the modular component to be easily grasped and manipulated during the assembly or disassembly of the core 405 and modular component 810. Portions of the upper and lower surfaces of the core 405 are exposed so that the core 405 can be grasped. The modular component can also be designed to be easily manipulated. According to one embodiment, the surfaces may be textured or contoured to provide additional friction between the surface and the user's fingers.

FIG. 8C shows an illustrative embodiment of the headpiece 800 in which the modular component 810 may be provided with a hinge 825, which allows a latching component 830 to open, thereby releasing the core 405.

According to one embodiment, one or more indicators may be present on the modular component 810 or a surface of the core 405. By way of example and not limitation, the indicators may include light emitting diodes 815 which indicate the battery condition. For example, as the battery discharges, a light emitting diode is illuminated to indicate the need to recharge or replace the battery. This could be advantageous for a parent or teacher who can visually determine the battery level.

Similarly, the core unit may have one or more visual elements which indicate the state of the cochlear implant. For example, a light emitting diode could have a first color and illumination pattern which indicates that cochlear implant is operational. The light emitting diode could have a different color and/or illumination pattern for various malfunctions such as a malfunction of the core, lack of communication between the core and antenna, or an internal processor malfunction.

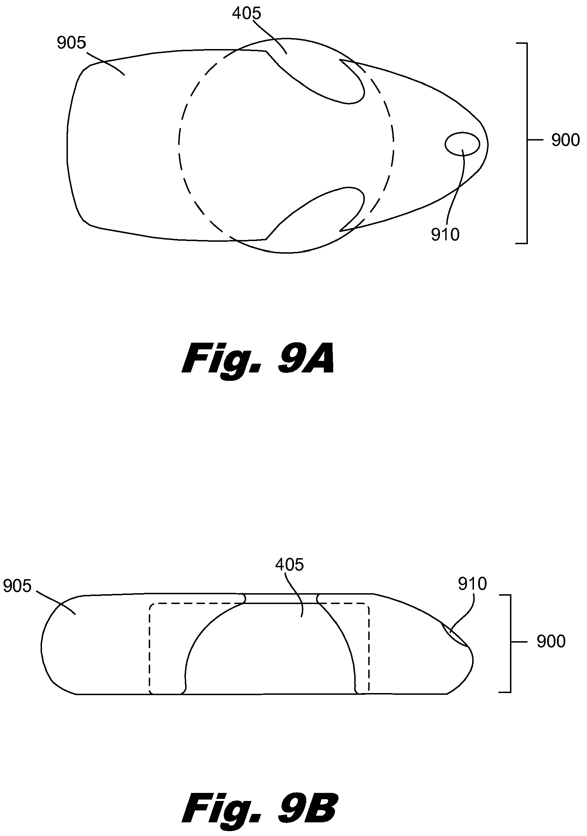

FIGS. 9A and 9B are a top view and a side view, respectively, of a modular speech processor headpiece 800 with an alternative geometry. In this embodiment, the core 405 is engaged on two sides by a modular component 905. The modular component 405 may attach in a variety of ways. For example, the core 405 may be simply snapped into the center of the modular component 905. In some illustrative embodiments, the core 405 may be keyed to ensure that the core 405 has the correct orientation with respect to the modular component 905.

To remove the core 405, the modular component 905 may be grasped in one hand and the core 405 removed with the opposite hand. The exposed portions of core 405 allow the user to grasp the core 405 by the sides and/or top and bottom of the core 405. As mentioned above, the core surfaces may be textured and/or contoured to provide a better gripping surface.

The modular component 905 may be balanced such that the center of mass of the modular component 905 aligns with the center of mass of the core 405. By aligning the center of mass of the modular component 905 with the center of mass of the core 405, the tendency for the headpiece 900 to rotate during acceleration or vibration can be reduced.

As previously discussed, a number of receivers, microphones, and battery technologies could be incorporated into the modular component 905. The modular component 905 and/or core 405 may contain a number of features including microphone ports, visual indicators, and other features. For example, although an omnidirectional microphone is generally preferred, the modular component 905 could additionally contain a directional microphone 910. The directional microphone 910 can be used by the patient to selectively amplify selected sound sources and to reduce undesirable background noise. According to one embodiment, the directional microphone 910 may be pointing in the same direction the patient is looking. For example, a patient may simply turn his head toward one who is speaking to point the directional microphone 910 in the speaker's direction to preferentially sense his voice.

The modular speech processor headpiece can allow for any of a number of modular components to be attached to the processor. For example, the patient may have two or more modular components. While one modular component is providing power to the cochlear implant, the other modular component can be recharging its battery. Additionally or alternatively, the functionality provided by different modular components may be different. The user can select the modular component that is most appropriate for the situation. For example, during a social event, the user may select a modular component that is less obtrusive or complements other clothing accessories. During the course of a normal day, the user may select a modular component with a longer lifetime or with a needed receiver. For example, if the user attends school, the user may need a battery that can supply power throughout the school day and a receiver that can receive amplified/filtered signals from a wireless microphone worn by the teacher.

At least some embodiments of the present modular speech processor headpiece may be configured to direct magnetic flux associated with the telemetry magnetic field generated by the telemetry coil (e.g. coil 120) and/or the retention magnetic filed generated by a retention magnet away from certain circuitry.

Turning to FIGS. 10A and 10B, the modular speech processor headpiece 500a illustrated therein includes a core 405a and the above-described modular ring 510. The core 405a is substantially similar to the core 405. Here, however, the headpiece is configured to direct magnetic flux away from electronic circuitry 464 (such as the sound processor 106, or at least a portion thereof) carried on the circuit board 462. The magnetic flux direction aspects associated with the modular speech processor headpiece 500a are applicable to the other headpieces described herein.

In the illustrated embodiment, the bottom surface 466 of the circuit board 462 may face generally towards the headpiece base plate 468. The electronic circuitry 464 may be configured to direct the implantable cochlear stimulator 110 to generate and apply electrical stimulation to one or more stimulation sites within the cochlea of a patient by transmitting control parameters (including, but not limited to, stimulation parameters) to the implantable cochlear stimulation device 104 via communications link 118. The electronic circuitry 464 may additionally or alternatively be configured to transmit power to the implantable cochlear stimulation device 104 and may be configured to receive data from the cochlear stimulation device 104.

The induction coil 120 in the exemplary core 405a illustrated in FIGS. 10A and 10B is disposed below the bottom surface 466 of the circuit board 462. The induction coil 120 may include a metallic wire or tube wound in a coiled configuration. In some examples, the induction coil 120 may include a coiled wire arranged in a generally disc-shaped and/or annular-shaped holder. It will be recognized that the induction coil 120 may have any suitable size and shape as may serve a particular application. The induction coil 120 may have a top surface 469 and an interior radial surface 470, as illustrated in FIG. 10A. The induction coil 120 may be seated in the headpiece base plate 468. Accordingly, the induction coil 120 may be in close proximity to the head of the patient when the headpiece base plate 468 is adjacent to the head.

With respect to magnetic flux direction, the exemplary core 405a includes a telemetry flux guide 472 positioned or disposed between the circuit board 462 and the induction coil 120. The telemetry flux guide 472 may have a generally annular shape with an inner radial surface 474 defining an aperture extending through a central portion of the telemetry flux guide 472. The telemetry flux guide may be adjacent to the bottom surface 466 of the circuit board 462 and the top surface 469 of the induction coil 120.

The exemplary telemetry flux guide 472 may include any material suitable for directing magnetic flux away from the circuit board 462. For example, the telemetry flux guide 472 may include a material having a relatively high resistivity that provides a low reluctance path for magnetic flux of the telemetry magnetic field. Additionally, the telemetry flux guide 472 may include a powdered material, such as a powdered metallic material having a relatively small particle size, in order to prevent the generation of eddy current in the telemetry flux guide 472. For example, eddy currents might be generated in a solid conductive material (as opposed to a powdered conductive material) in the presence of the telemetry magnetic field since the telemetry magnetic field is a changing magnetic field generated by an alternating current passing through the induction coil 120.

The powdered material in the exemplary telemetry flux guide 472 may be held together using any suitable material, such as a polymer material. In some examples, the powdered metallic material may include iron and/or other ferrite materials. Additionally, the telemetry flux guide 472 may be suitable for frequencies of telemetry signals generated and/or received by the induction coil 120, such as, for example, an approximately 49 MHz telemetry signal and/or an approximately 10.7 MHz telemetry signal. A telemetry flux guide 472 including a material having a relative permeability (*i.e.,* the ratio of the permeability of the alloy to the permeability of free-space) of approximately 9 may be suitable for a frequency range that includes 49 MHz and 10.7 MHz telemetry signals. However, it will be recognized that the telemetry flux guide 472 may have any other suitable relative permeability value as may serve a particular application. Additionally or alternatively, telemetry flux guide 472 may have a relatively high resistivity and a relatively small particle size in order to facilitate redirection of magnetic flux while minimizing the generation of eddy currents in the telemetry flux guide 472.

The telemetry flux guide 472 may be positioned and configured to direct magnetic flux of the magnetic field generated by the induction coil 120 away from the circuit board 462, as will be described in greater detail below. For example, a telemetry magnetic field may generated by the induction coil 120 to transmit a telemetry signal. Magnetic flux of the telemetry magnetic field may be directed away from the circuit board 462 by the telemetry flux guide 472, thereby protecting the electronic circuitry 464 on the circuit board 462 from the telemetry magnetic field. By directing the magnetic flux in the telemetry magnetic field away from the circuit board 462, energy losses from the induction coil 120 to the electronic circuitry 464 via the telemetry magnetic field may be minimized, thereby extending the life of batteries used to provide power to one or more components of the cochlear implant system.

In some examples, the core 405a may additionally or alternatively include a retention magnet 476 disposed between the circuit board 462 and the headpiece base plate 468. The retention magnet 476 may have a top surface 478 generally facing the bottom surface 466 of the circuit board 462. The retention magnet 476 may additionally have an outer radial surface 480. In some embodiments, the retention magnet 476 may be positioned in the core 405a such that the retention magnet 476 is at least partially surrounded by the induction coil 120 and/or the telemetry flux guide 472. Accordingly, the outer radial surface 480 of the retention magnet 476 may generally face the inner radial surface 470 of the induction coil 120 and/or the inner radial surface 474 of the telemetry flux guide 472. For example, the retention magnet 476 may be positioned in an aperture defined by the inner radial surface 474 extending through the telemetry flux guide 472.

The retention magnet 476 may be configured to produce a retention magnetic field for securing one or more components of a cochlear implant system the head of the patient. For example, the retention magnet 476 may be disposed adjacent to the core base plate 468 such that the retention magnet 476 is in close proximity to the head of the patient when the core base plate 468 is adjacent to the head.

As noted above, the exemplary cochlear stimulation device 104 (FIG. 6) in the cochlear implant system 100 may include a magnet 123 configured to magnetically couple with the retention magnet 476. Accordingly, when the core base plate 468 is positioned adjacent to the head of the patient near the coil 122, the retention magnet 476 may be magnetically coupled to the magnet of the cochlear stimulation portion 104, thereby securing and/or orienting the headpiece 500a on the head.

The core 405a in the exemplary headpiece 500a may additionally or alternatively include a retention flux guide 482 positioned between the circuit board 462 and the retention magnet 476. The retention flux guide 482 may at least partially surround the retention magnet 476. As illustrated in FIGS. 10A and 10B, a top wall 484 of the retention flux guide 482 may be disposed in between and adjacent to the bottom surface 466 of the circuit board 462 and the top surface 478 of the retention magnet 476. Additionally, a side wall 486 of the retention flux guide 482 may at least partially surround the outer radial surface 480 of the retention magnet 476. The side wall 486 of the retention flux guide 482 may be adjacent to the outer radial surface 480 of the retention magnet 476, the inner radial surface 470 of the induction coil 120, and/or the inner radial surface 474 of the telemetry flux guide 472.

The retention flux guide 482 may include any material suitable for redirecting magnetic flux associated with a magnetic field produced by the retention magnet 476. For example, the retention flux guide 482 may include a material having a relatively high permeability. In some examples, the retention flux guide 482 may include a metallic material, such as a mu-metal alloy comprising nickel and iron. A relatively high permeability mu-metal alloy may have a relative permeability between approximately 60,000 and 300,000. For example, a mu-metal alloy may have a relative permeability of approximately 100,000. A high-permeability mu-metal alloy may include any suitable ratio of nickel and iron, such as, for example, a ratio of approximately 80% nickel and 20% iron. It will be recognized that the retention flux guide 482 may alternatively include any suitable material having any suitable relative permeability.

The retention flux guide 482 may be configured to direct magnetic flux of a retention magnetic field surrounding the retention magnet 476 away from the circuit board 462 and/or away from the telemetry flux guide 472. Accordingly, magnetic flux from the retention magnet 476 may be directed away from the circuit board 462, thereby protecting the electronic circuitry 464 on the circuit board 462 from the retention magnetic field.

As mentioned, the retention flux guide 482 may also direct magnetic flux of the retention magnetic field away from the telemetry flux guide 472, thereby preventing saturation of powdered metallic material in the telemetry flux guide 472 with magnetic flux from the retention magnet. Magnetic flux from the retention magnet 476 may significantly reduce the relative permeability of the powdered metallic material in the telemetry flux guide 472, reducing the effectiveness of the telemetry flux guide 472 in directing magnetic flux from the induction coil 120 away from the circuit board 462. Accordingly, the retention flux guide 482 may direct magnetic flux of the retention magnetic field away from the telemetry flux guide 472, thereby preventing magnetic flux from saturating the telemetry flux guide 472.

By directing magnetic flux away from the electronic circuitry 464 in the circuit board 462, the telemetry flux guide 472 and/or the retention flux guide 482 may enable the induction coil 120 and/or the retention magnet 476 to be located within the headpiece core 405a in relatively close proximity to the circuit board 462. Accordingly, the speech processor headpiece 500a may be made more compact by consolidating electronic and magnetic field emitting components within a portion of the headpiece, as illustrated in FIGS. 10A and 10B. This may increase the ease of use and comfort for a patient using the cochlear implant system in comparison to a larger modular unit or a conventional cochlear implant systems (e.g. a BTE system) in which electronic circuitry is separated from the headpiece.

In some cochlear implant system embodiments, the cochlear stimulation device 104 may additionally or alternatively include a retention flux guide and/or a telemetry flux guide for directing magnetic flux away from electronic components in the cochlear stimulation device 104. For example, in the cochlear stimulation device that includes an induction coil and/or a retention magnet (e.g. device 104 in FIG. 6), a retention flux guide and/or a telemetry flux guide may be included in the cochlear stimulation device to redirect magnetic flux away from electronic circuitry that may be located in close proximity to the induction coil and/or the retention magnet.

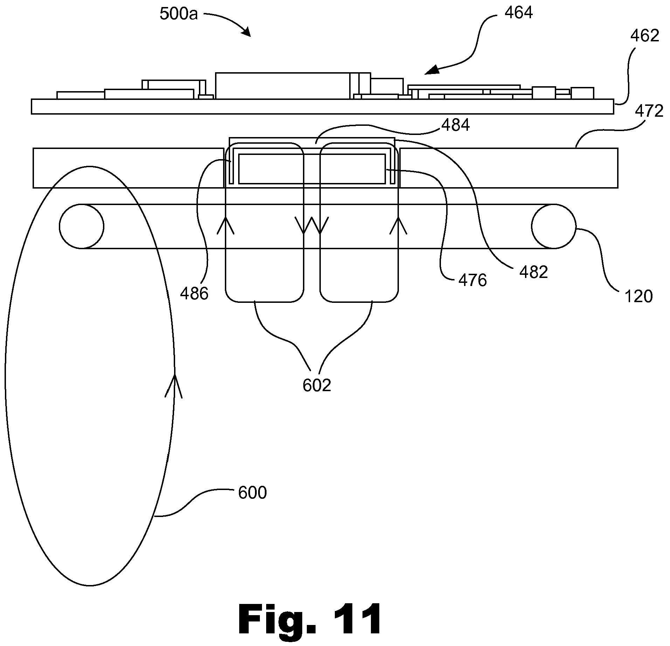

FIG. 11 illustrates magnetic flux surrounding an induction coil 120 and a retention magnet 476 in the exemplary headpiece 500a in accordance with the present systems and methods. As illustrated in FIG. 11, magnetic flux 600 may surround the induction coil 120. The magnetic flux 600 is represented as a flux path surrounding the induction coil 120. Additionally, magnetic flux 602 may pass through and surround retention magnet 476. The magnetic flux 602 is represented as flux paths surrounding and passing through the retention magnet 476. It will be recognized that additional flux paths other than those illustrated in FIG. 11 may be associated with the telemetry magnetic field surrounding the induction coil 120 and the retention magnetic field surrounding the retention magnet 476.

The telemetry flux guide 472 may provide a low reluctance path for the magnetic flux 600 surrounding the induction coil 120. As illustrated in FIG. 11, the path of the magnetic flux 600 may be directed through the telemetry flux guide 472 such that a path of the magnetic flux 600 between the induction coil 120 and the circuit board 462 may be shortened. The magnetic flux 600 of the telemetry magnetic flux field surrounding the induction coil 120 may therefore be redirected by the telemetry flux guide 472 such that the magnetic flux 600 is substantially prevented from reaching the circuit board 462, thereby reducing or eliminating magnetic flux passing through the electronic circuitry 464.

The retention flux guide 482 may provide a high permeability path for the magnetic flux 602 surrounding and passing through the retention magnet 476. As illustrated in FIG. 11, the path of the magnetic flux 602 may be directed through the retention flux guide 482 such that a path of the magnetic flux 602 passes generally through and/or along the top wall 484 and/or the side wall 486 of the retention flux guide 482. Accordingly, a path of the magnetic flux 602 between the induction coil 120 and the circuit board 462 may be shortened. Similarly, a path of the magnetic flux 602 between the retention magnet 476 and the telemetry flux guide 472 may be shortened. The magnetic flux 602 of the retention magnetic flux field surrounding and passing through the retention magnet 476 may therefore be redirected by the retention flux guide 482 such that the magnetic flux 602 is substantially prevented from reaching the circuit board 462 and/or the telemetry flux guide 472, thereby reducing or eliminating magnetic flux from the retention magnet passing through the electronic circuitry 464 and/or the telemetry flux guide 472.

In sum, a modular speech processor headpiece combines the external components of the cochlear implant system into a single unit that is worn directly over the surgically implanted antenna. This eliminates the need for a separate body worn processor or BTE processor and the connecting cable. Consequently, the modular speech processor headpiece reduces the complexity of wearing and using a cochlear implant. The modular speech processor headpiece eliminates the need to route a cable through clothing or hair and the possibility of snagging a cable. Additionally, the modular speech processor headpiece can be more robust, modular, and significantly less visually intrusive than processors of conventional cochlear implant systems.

The preceding description has been presented only to illustrate and describe embodiments and examples of the principles described. This description is not intended to be exhaustive or to limit these principles to any precise form disclosed. Many modifications and variations are possible within the scope of the appended claims.

## Claims

1. A cochlear implant system, comprising:
a cochlear implant (104) comprising:
an electrode array (112, 114) adapted to be implanted within a cochlea,
an internal processor (106) in communication with the electrode array, and
an antenna (122) electrically coupled to the internal processor; and
an external headpiece (102, 400, 500, 500a, 700, 800, 900) configured as a stand alone, head-mounted unit which provides external functionality for operation of the cochlear implant system, wherein at least one of the cochlear implant and the external headpiece includes a magnet (123, 476), the other of the cochlear implant and the external headpiece includes a magnet (123, 476) or magnetic material, and the magnetic attraction associated therewith allows the external headpiece to be removably positioned over the antenna such that a portion of skin is sandwiched between the antenna and the external headpiece,
**characterised in that**:
the external head piece is a modular external headpiece comprising:
a core (405, 405a) containing a sound processor for processing sound and providing a corresponding signal to the antenna, the core comprising a lower surface that faces the cochlear implant and an upper surface (435) that faces in a direction opposite the lower surface, and
a modular component (410, 510, 710, 810, 905) configured to releasably engage the core and supply electrical power to the core;
the core and the modular component being respectively configured such that, when the modular component engages the core to form the modular external headpiece, the modular component is configured to at least partially enclose the perimeter of the core and to leave at least a portion of the upper surface and the lower surface of the core exposed.

2. The cochlear implant system of claim 1, wherein the sound processor (106) of the core comprises a microphone, signal processing electronics, and a transmitter, the core being configured to convert sound into electrical signals using the microphone, manipulate the electrical signals using the signal processing electronics to produce modified electrical signals, and transmit the modified electrical signals to the antenna using the transmitter.

3. The cochlear implant system of claim 1, wherein the modular component (410, 510, 710) is a modular ring entirely circumscribing the core when the modular ring is attached to the core

4. The cochlear implant system of claim 3, wherein the core (405, 405a) further comprises a substantially cylindrical shape having a plurality of slots (420) disposed around an outer perimeter; and wherein the modular ring (410, 510, 710) has a number of tabs (445) configured to be received by the plurality of slots such that the modular ring is securely fastened to the core.

5. The cochlear implant system of claim 1, wherein the core (405, 405a) further comprises a number of electrical contacts (430) disposed about an exterior surface, and wherein the modular component (410, 510, 710, 810, 905) further comprises a number of electrical contacts (450) disposed about an inner surface such that electrical contact is established between the modular component and the core when the modular component engages the core.

6. The cochlear implant system of claim 1, further comprising a seal (455) disposed between the core (405, 405a) and the modular component such that an electrical connection between the core and the modular component is sealed when the modular component engages the core.

7. The cochlear implant system of claim 1, wherein the modular component (410) contains a wired interface (460).

8. The cochlear implant system of claim 1, wherein the modular component (410, 510, 810, 905) comprises a wireless receiver and/or a microphone.

9. The cochlear implant system of claim 1, wherein
the modular external headpiece core (405a) includes an induction coil (120) configured to transmit a telemetry signal by generating a telemetry magnetic field and a telemetry flux guide (472) positioned between the induction coil and the sound processor; and
the telemetry flux guide is configured to direct magnetic flux of the telemetry magnetic field away from the sound processor.

10. The cochlear implant system of claim 1, wherein
the modular external headpiece core (405a) includes a retention magnet (476) configured to produce a retention magnetic field for securing one or more components of the cochlear implant system to a head of the patient and a retention flux guide (482) positioned between the retention magnet and the sound processor; and
wherein said retention flux guide is configured to direct magnetic flux of the retention magnetic field away from the sound processor.

11. A cochlear implant system comprising a cochlear implant and a modular external headpiece, the modular external headpiece comprising:
a core (405, 405a) comprising:
a lower surface that faces the cochlear implant and an upper surface (435) that faces in a direction opposite the lower surface,
a microphone and sound processor for producing a signal representing ambient sound to be transmitted to a cochlear implant, and
a first plurality of electrical contacts (430); and
a plurality of modular components (410, 510, 710, 810, 905) in a set, wherein each of the modular components in the set comprises a second plurality of electrical contacts (450);
wherein each of the modular components is configured to be individually mounted onto the core such that electrical communication is made between a portion of the first plurality of electrical contacts and at least a portion of the second plurality of electrical contacts;
**characterized in that**
the core and each of the modular components are respectively configured such that, when a single one of the modular components engages the core to form the modular external headpiece, the modular component is configured to at least partially enclose the perimeter of the core and to leave at least a portion of the upper surface and the lower surface of the core exposed; and
wherein at least one of the cochlear implant and the modular external headpiece includes a magnet (123, 476), the other of the cochlear implant and the modular external headpiece includes a magnet (123, 476) or magnetic material, and the magnetic attraction associated therewith allows the modular external headpiece to be removably positioned overt an antenna of the cochlear implant such that a portion of skin is sandwiched between the antenna and the modular external headpiece.

12. The cochlear implant system of claim 11, wherein each of the modular components (410, 510, 710, 810, 905) is configured to expand the functionality of the cochlear implant system by at least one of: supplying battery power to the core, receiving wireless signals, supplying additional processing capability, supplying additional programming capability, providing an interface for a wired connection of external devices to the cochlear implant system, and changing the external appearance of the cochlear implant system.

13. The cochlear implant system of claim 11, wherein the core (405, 405a) comprises a cylindrical shape and each of the modular component (410, 510, 710) has an annular shape configured to receive the cylindrical core in a center thereof while allowing access to the top and the bottom of the core.

14. The cochlear implant system of claim 11, wherein
one of the plurality of modular components within the set comprises a wired connection (460) to a computer, the computer being configured to program the sound processor in the core; or
at least one of the plurality of modular components within the set comprises a waterproof seal (455) between the core and modular component when engaged; or
at least one of the plurality of modular components within the set comprises a receiver for receiving an electronic signal from an external device.

15. The cochlear implant system of claim 11, wherein
the core (405a) includes an induction coil (120) configured to transmit a telemetry signal by generating a telemetry magnetic field and a telemetry flux guide (472) positioned between the induction coil and the sound processor; and
the telemetry flux guide is configured to direct magnetic flux of the telemetry magnetic field away from the sound processor.

16. The cochlear implant system of claim 11, wherein
the core (405a) includes a retention magnet (476) configured to produce a retention magnetic field for securing one or more components of the cochlear implant system to a head of the patient and a retention flux guide (482) positioned between the retention magnet and the sound processor; and
wherein said retention flux guide is configured to direct magnetic flux of the retention magnetic field away from the sound processor.

## Patentansprüche

1. Cochlearimplantatsystem mit:
einem Cochlearimplantat (104) mit:
einer Elektrodenanordnung (112, 114) zum implantieren innerhalb einer Cochlea,
einem internen Prozessor (106) in Verbindung mit der Elektrodenanordnung, und
einer Antenne (122), die elektrisch mit dem internen Prozessor gekoppelt ist; und
einem externen Kopfstück (102, 400, 500, 500a, 700, 800, 900), welches als alleinstehende, am Kopf montierte Einheit ausgebildet ist, die externe Funktionalität für den Betrieb des Cochlearimplantatsystems liefert, wobei das Cochlearimplantat und/oder das externe Kopfstück einen Magneten (123, 476) aufweist bzw. aufweisen, wobei das Gegenstück zu dem Cochlearimplantat bzw. dem externen Kopfstück einen Magneten (123, 476) oder magnetisches Material aufweist, und wobei die damit assoziierte magnetische Anziehung eine lösbare Positionierung des externen Kopfstücks über der Antenne erlaubt, so dass ein Teil der Haut zwischen der Antenne und dem externen Kopfstück in sandwichartiger Weise angeordnet ist,
**dadurch gekennzeichnet, dass**:
es sich bei dem externen Kopfstück um ein modulares externes Kopfstück handelt, mit:
einem Kern (405, 405a), der einen Schallprozessor zum Verarbeiten von Schall und zum Liefern eines entsprechenden Signals an die Antenne aufweist, wobei der Kern eine untere Fläche, die dem Cochlearimplantat zugewandt ist, und eine obere Fläche (435) aufweist, die in eine Richtung entgegengesetzt zur unteren Fläche gewandt ist, und
einer modufaren Komponente (410, 510, 710, 810, 905) zum lösbaren Eingriff mit dem Kern und zum Zuführen von elektrischer Leistung zu dem Kern;
wobei der Kern und die modulare Komponente jeweils so konfiguriert sind, dass, wenn die modulare Komponente mit dem Kern in Eingriff steht, um das modulare externen Kopfstück zu bilden, die modulare Komponente ausgebildet ist, um den Umfang des Kerns mindestens zum Teil zu umschließen und mindestens einen Abschnitt der oberen Fläche und der unteren Fläche des Kerns frei zu lassen.

2. Cochlearimplantatsystem gemäß Anspruch 1, wobei der Schallprozessor (106) des Kerns ein Mikrofon, Signalverarbeitungselektronik und einen Sender aufweist, und wobei der Kern ausgebildet ist, um Schall in elektrische Signale unter Verwendung des Mikrofons umzuwandeln, die elektrischen Signale unter Verwendung der Signalverarbeitungselektronik zu manipulieren, um modifizierte elektrische Signale zu erzeugen, und die modifizierten elektrischen Signale unter Verwendung des Senders an die Antenne zu senden.

3. Cochlearimplantatsystem gemäß Anspruch 1, wobei es sich bei der modularen Komponente (410, 510, 710) um einen modularen Ring handelt, der den Kern vollständig umschreibt, wenn der modulare Ring an dem Kern befestigt ist.

4. Cochlearimplantatsystem gemäß Anspruch 3, wobei der Kern (405, 405a) ferner eine im Wesentlichen zylindrische Form mit einer Mehrzahl von Schlitzen (420) aufweist, die um einen Außenumfang herum angeordnet sind; und wobei der modulare Ring (410, 510, 710) eine Anzahl von Vorsprüngen (445) aufweist, die ausgebildet sind, um von der Mehrzahl von Schlitzen so aufgenommen zu werden, so dass der modulare Ring sicher an dem Kern befestigt ist.

5. Cochlearimplantatsystem gemäß Anspruch 1, wobei der Kern (405, 405a) ferner eine Anzahl von elektrischen Kontakten (430) aufweist, die auf einer Außenfläche angeordnet sind, und wobei die modulare Komponente (410, 510, 710, 810, 905) ferner eine Anzahl von elektrischen Kontakten (450) aufweist, die so auf einer Innenfläche angeordnet sind, dass ein elektrischer Kontakt zwischen der modularen Komponente und dem Kern hergestellt wird, wenn die modulare Komponente in Eingriff mit dem Kern tritt.

6. Cochlearimplantatsystem gemäß Anspruch 1, ferner versehen mit einer Dichtung (455), die so zwischen dem Kern (405, 405a) und der modularen Komponente angeordnet ist, dass eine elektrische Verbindung zwischen dem Kern und der modularen Komponente abgedichtet wird, wenn die modulare Komponente mit dem Kern in Eingriff steht.

7. Cochlearimplantatsystem gemäß Anspruch 1, wobei die modulare Komponente (410) eine drahtgebundene Schnittstelle (460) aufweist.

8. Cochlearimplantatsystem gemäß Anspruch 1, wobei die modulare Komponente (410, 510, 810, 905) einen drahtlosen Empfänger und/oder ein Mikrofon aufweist.

9. Cochlearimplantatsystem gemäß Anspruch 1, wobei,
der Kern (405a) des modularen externen Kopfstücks eine Induktionsspule (120) zum Senden eines Telemetriesignals durch Erzeugen eines Telemetriemagnetfelds und eine Telemetrieflussführung (472) aufweist, die zwischen der Induktionsspule und dem Schallprozessor angeordnet ist; und
die Telemetrieflussführung ausgebildet ist, um magnetischen Fluss des Telemetriefelds von dem Schallprozessor weg zu lenken,

10. Cochlearimplantatsystem gemäß Anspruch 1, wobei
der Kern (405a) des modularen externen Kopfstücks einen Haltemagnet (476) zum Erzeugen eines Haftemagnetfelds zum Befestigen einer oder mehrerer Komponenten des Cochlearimplantatsystems am Kopf des Patienten und eine Halteflussführung (482) beinhaltet, die zwischen dem Haltemagnet und dem Schallprozessor angeordnet ist; und
wobei die Halteflussführung ausgebildet ist, um magnetischen Fluss des Haltemagnetfelds weg von dem Schallprozessor zu lenken.

11. Cochlearimplantatsystem mit einem Cochlearimplantat und einem modularen externen Kopfstück, welches versehen ist mit:
einem Kern (405, 405a) mit:
einer unteren Fläche, die dem Cochlearimplantat zugewandt ist, und einer oberen Fläche (435), welche in eine Richtung entgegengesetzt der unteren Fläche gewandt ist,
einem Mikrofon und einem Schallprozessor zum Erzeugen eines Umgebungsschall-repräsentativen Signals, welches zu einem Cochlearimplantat zu senden ist, und
einer ersten Mehrzahl von elektrischen Kontakten (430); und
einer Mehrzahl von modularen Komponenten (410, 510, 710, 810, 905) in einem Satz, wobei jede der modularen Komponenten in dem Satz eine zweite Mehrzahl von elektrischen Kontakten (450) aufweist;
wobei jede der molaren Komponenten ausgebildet ist, um individuell auf dem Kern angebracht zu werden, so dass elektrische Kommunikation zwischen einem Teil der ersten Mehrzahl von elektrischen Kontakten und mindestens einem Teil der zweiten Mehrzahl von elektrischen Kontakten hergestellt wird;
**dadurch gekennzeichnet, dass**
der Kern und jede der modularen Komponenten so konfiguriert sind, dass, wenn eine einzelne der modularen Komponenten in Eingriff mit dem Kern steht, um das modulare externe Kopfstück zu bilden, die modulare Komponente ausgebildet ist, um den Umfang des Kerns mindestens zum Teil zu umschließen und mindestens einen Teil der oberen Fläche und der unteren Fläche des Kerns freizulassen; und
wobei das Cochlearimplantat und/oder das modulare externe Kopfstück einen Magneten (123, 476) aufweisen, wobei das Gegenstück des Cochlearimplantats bzw. des modularen externen Kopfstücks einen Magneten (123, 476) oder magnetisches Material aufweist, und wobei die damit assoziierte magnetische Anziehung eine lösbare Positionierung des modularen externen Kopfstücks über einer Antenne des Cochlearimplantats in einer Weise ermöglicht, dass ein Abschnitt der Haut sandwichartig zwischen der Antenne und dem modularen externen Kopfstück liegt.

12. Cochlearimplantatsystem gemäß Anspruch 11, wobei jede der modularen Komponenten (410, 510, 710, 810, 905) konfiguriert ist, um die Funktionalität des Cochlearimplantatsystems durch mindestens eine der folgenden Funktionen zu erweitern: Versorgung des Kerns mit Batterieleistung, Empfangen von drahtlosen Signalen, Bereitstellen von zusätzlicher Verarbeitungskapazität, Bereitstellen von zusätzlicher Programmierkapazität, Bereitstellen einer Schnittstelle für eine drahtgebundene Verbindung eines externen Geräts mit dem Cochlearimplantatsystem, und Veränderung der externen Erscheinung des Cochlearimplantatsystems.

13. Cochlearimplantatsystem gemäß Anspruch 11, wobei der Kern (405, 405a) eine zylindrische Form aufweist und jede der modularen Komponenten (410, 510, 710) eine ringförmige Form aufweist, die ausgebildet ist, um den zylindrischen Kern im Zentrum aufzunehmen, während Zugang zu dem oberen Ende und dem unteren Ende des Kerns gewährt wird.

14. Cochlearimplantatsystem gemäß Anspruch 11, wobei
eine der Mehrzahl der modularen Komponenten innerhalb des Satzes eine drahtgebundene Verbindung (460) zu einem Computer aufweist, welcher ausgebildet ist, um den Schallprozessor in dem Kern zu programmieren; oder
mindestens eine der Mehrzahl von modularen Komponenten innerhalb des Satzes eine wasserdichte Dichtung (455) zwischen dem Kern und der modularen Komponente bei Eingriff aufweist; oder
mindestens eine der Mehrzahl von modularen Komponenten innerhalb des Satzes einen Empfänger zum Empfangen eines elektronischen Signals von einem externen Gerät aufweist.

15. Cochlearimplantatsystem gemäß Anspruch 11, wobei
der Kern (405a) eine Induktionsspule (120), die zum Senden eines Telemetriesignals mittels Erzeugen eines Telemetriemagnetfelds ausgebildet ist, und eine Telemetrieflussführung (472) aufweist, die zwischen der Induktionsspule und dem Schallprozessor angeordnet ist; und
die Telemetrieflussführung ausgebildet ist, um magnetischen Fluss des Telemetriemagnetfelds von dem Schallprozessor weg zu lenken.

16. Cochlearimplantatsystem gemäß Anspruch 11, wobei
der Kern (405a) einen Haltemagneten (476), der konfiguriert ist, um ein Haltemagnetfeld zum Befestigen einer oder mehrerer Komponenten des Copchlearimplantatsystems am Kopf des Patienten zu erzeugen, und eine Halteflussführung (482) aufweist, die zwischen dem Haltemagneten und dem Schallprozessor angeordnet ist; und
die Halteflussführung ausgebildet ist, um magnetischen Fluss des Haltemagnetfelds von dem Schallprozessor weg zu lenken.

## Revendications

1. Système d'implant cochléaire, comprenant :
un implant cochléaire (104) comprenant :
un réseau d'électrodes (112, 114) conçu pour être implanté au sein d'une cochlée ;
un processeur interne (106) en communication avec le réseau d'électrodes ;
une antenne (122) couplée électriquement au processeur interne ; et
une oreillette externe (102, 400, 500, 500a, 700, 800, 900) configurée sous forme d'une unité autonome, montée au niveau de la tête, qui fournit une fonctionnalité externe pour le fonctionnement du système d'implant cochléaire, où au moins un dispositif parmi l'implant cochléaire et l'oreillette externe comprend un aimant (123, 476), l'autre dispositif parmi l'implant cochléaire et l'oreillette externe comprend un aimant (123, 476) ou un matériau magnétique, et l'attraction magnétique associée à ceux-ci permet à l'oreillette externe d'être positionnée de manière amovible sur l'antenne de manière à ce qu'une partie de peau soit prise en sandwich entre l'antenne et l'oreillette externe,
**caractérisé en ce que** :
l'oreillette externe est une oreillette externe modulaire comprenant :
un noyau (405, 405a) contenant un processeur de son pour traiter le son et fournir un signal correspondant à l'antenne, le noyau comprenant une surface inférieure qui fait face à l'implant cochléaire et une surface supérieure (435) qui fait face à une direction opposée à la surface inférieure, et
un composant modulaire (410, 510, 710, 810, 905) configuré pour accueillir de manière amovible le noyau et alimenter en énergie électrique le noyau ;
le noyau et le composant modulaire étant respectivement configurés de manière à ce que, lorsque le composant modulaire accueille le noyau pour former l'oreillette externe modulaire, le composant modulaire est configuré pour enclore, au moins partiellement, le périmètre du noyau et pour laisser au moins une partie de la surface supérieure et de la surface inférieure du noyau exposée.

2. Système d'implant cochléaire selon la revendication 1, dans lequel le processeur de son (106) du noyau comprend un microphone, des circuits électroniques de traitement du signal, et un émetteur, le noyau étant configuré pour convertir le son en signaux électriques à l'aide du microphone, pour manipuler les signaux électriques au moyen des circuits électroniques de traitement du signal pour produire des signaux électriques modifiés, et pour transmettre les signaux électriques modifiés à l'antenne au moyen de l'émetteur.

3. Système d'implant cochléaire selon la revendication 1, dans lequel le composant modulaire (410, 510, 710) est un anneau modulaire entourant entièrement le noyau lorsque l'anneau modulaire est fixé au noyau.

4. Système d'implant cochléaire selon la revendication 3, dans lequel le noyau (405, 405a) comprend en outre une forme sensiblement cylindrique ayant une pluralité de fentes (420) disposées autour d'un périmètre extérieur ; et où l'anneau modulaire (410, 510, 710) comporte un certain nombre de languettes (445) configurées pour être reçues par la pluralité de fentes de sorte que l'anneau modulaire soit solidement fixé au noyau.

5. Système d'implant cochléaire selon la revendication 1, dans lequel le noyau (405, 405a) comprend en outre un certain nombre de contacts électriques (430) disposés autour d'une surface extérieure, et où le composant modulaire (410, 510, 710, 810, 905) comprend en outre un certain nombre de contacts électriques (450) disposés autour d'une surface intérieure de sorte qu'un contact électrique soit établi entre le composant modulaire et le noyau lorsque le composant modulaire accueille le noyau.

6. Système d'implant cochléaire selon la revendication 1, comprenant en outre un joint (455) disposé entre le noyau (405, 405a) et le composant modulaire de sorte qu'une connexion électrique entre le noyau et le composant modulaire soit scellée lorsque l'élément modulaire accueille le noyau.

7. Système d'implant cochléaire selon la revendication 1, dans lequel le composant modulaire (410) contient une interface câblée (460).

8. Système d'implant cochléaire selon la revendication 1, dans lequel le composant modulaire (410, 510, 810, 905) comprend un récepteur sans fil et/ou un microphone.

9. Système d'implant cochléaire selon la revendication 1, dans lequel
le noyau de l'oreillette externe modulaire (405a) comprend une bobine d'induction (120) configurée pour transmettre un signal de télémétrie en générant un champ magnétique de télémesure et un guide de flux de télémesure (472) positionné entre la bobine d'induction et le processeur de son ; et
le guide de flux de télémesure est configurée pour diriger le flux magnétique du champ magnétique de télémesure hors du processeur de son.

10. Système d'implant cochléaire selon la revendication 1, dans lequel
le noyau de l'oreillette externe modulaire (405a) comprend un aimant de retenue (476) configuré pour produire un champ magnétique de retenue pour fixer un ou plusieurs composants du système d'implant cochléaire au niveau de la tête du patient et un guide de flux de retenue (482) positionné entre l'aimant de retenue et le processeur de son ; et
où ledit guide de flux de retenue est configuré pour diriger le flux magnétique du champ magnétique de retenue hors du processeur de son.

11. Système d'implant cochléaire comprenant un implant cochléaire et une oreillette externe modulaire, l'oreillette externe modulaire comprenant :
un noyau (405, 405a) comprenant :
une surface inférieure qui fait face à l'implant cochléaire et une surface supérieure (435) qui fait face à une direction opposée à la surface inférieure,
un microphone et un processeur de son pour produire un signal représentant le son ambiant devant être transmis à un implant cochléaire, et
une première pluralité de contacts électriques (430) ; et
une pluralité de composants modulaires (410, 510, 710, 810, 905) dans un ensemble, où chacun des composants modulaires contenus dans l'ensemble comprend une seconde pluralité de contacts électriques (450) ;
où chacun des éléments modulaires est configuré pour être monté individuellement sur le noyau de sorte qu'une communication électrique soit établie entre une partie de la première pluralité de contacts électriques et au moins une partie de la seconde pluralité de contacts électriques ;
**caractérisé en ce que**
le noyau et chacun des composants modulaires sont respectivement configurés de manière à ce que, lorsqu'un des composants modulaires accueille le noyau pour former l'oreillette externe modulaire, le composant modulaire est configuré pour enclore, au moins partiellement, le périmètre du noyau et pour laisser au moins une partie de la surface supérieure et de la surface inférieure du noyau exposée ; et
où au moins un dispositif parmi l'implant cochléaire et l'oreillette externe comprend un aimant (123, 476), l'autre dispositif parmi l'implant cochléaire et l'oreillette externe comprend un aimant (123, 476) ou un matériau magnétique, et l'attraction magnétique associée à ceux-ci permet à l'oreillette externe d'être positionnée de manière amovible sur une antenne de l'implant cochléaire de manière à ce qu'une partie de peau soit prise en sandwich entre l'antenne et l'oreillette externe modulaire.

12. Système d'implant cochléaire selon la revendication 11, dans lequel chacun des composants modulaires (410, 510, 710, 810, 905) est configuré pour étendre la fonctionnalité du système d'implant cochléaire par l'intermédiaire d'au moins une des actions suivantes : alimenter le noyau par batterie, recevoir des signaux sans fil, fournir une capacité de traitement supplémentaire, fournir une capacité de programmation supplémentaire, fournir une interface permettant la connexion par câble de dispositifs externes au système d'implant cochléaire, et changer l'aspect extérieur du système d'implant cochléaire.

13. Système d'implant cochléaire selon la revendication 11, dans lequel le noyau (405, 405a) comprend une forme cylindrique et chacun des éléments modulaires (410, 510, 710) présente une forme annulaire configurée pour recevoir le noyau cylindrique en son centre tout en permettant l'accès à la partie supérieure et à la partie inférieure du noyau.

14. Système d'implant cochléaire selon la revendication 11, dans lequel
un composant de la pluralité de composants modulaires au sein de l'ensemble comprend une connexion par câble (460) à un ordinateur, l'ordinateur étant configuré pour programmer le processeur de son dans le noyau ; ou au moins un composant de la pluralité de composants modulaires au sein de l'ensemble comprend un joint d'étanchéité à l'eau (455) entre le noyau et le composant modulaire lorsqu'il est accueilli ;
au moins un composant de la pluralité de composants modulaires au sein de l'ensemble comprend un récepteur destiné à recevoir un signal électronique provenant d'un dispositif externe.

15. Système d'implant cochléaire selon la revendication 1, dans lequel
le noyau (405a) comprend une bobine d'induction (120) configurée pour transmettre un signal de télémétrie en générant un champ magnétique de télémesure et un guide de flux de télémesure (472) positionné entre la bobine d'induction et le processeur de son ; et
le guide de flux de télémesure est configurée pour diriger le flux magnétique du champ magnétique de télémesure hors du processeur de son.

16. Système d'implant cochléaire selon la revendication 11, dans lequel
le noyau (405a) comprend un aimant de retenue (476) configuré pour produire un champ magnétique de retenue pour fixer un ou plusieurs composants du système d'implant cochléaire au niveau de la tête du patient et un guide de flux de retenue (482) positionné entre l'aimant de retenue et le processeur de son ; et
où ledit guide de flux de retenue est configuré pour diriger le flux magnétique du champ magnétique de retenue hors du processeur de son.
